# EUROPEAN PATENT APPLICATION

(11) **EP 4 265 737 A1**
(43) Date of publication of application: **25.10.2023**
(21) Application number: 22169338.5
(22) Date of filing: 21.04.2022
(51) Int. Cl.: C12Q 1/6883

(54) **METHYLATION MARKERS FOR PREDICTING SENSITIVITY TO TREATMENT WITH ANTIBODY BASED THERAPY**

(71) Applicant: Academisch Medisch Centrum, 1105 AZ Amsterdam (NL)
(72) Inventor: The designation of the inventor has not yet been filed
(74) Representative: de Pauw, Elmar Sebastian David

(57) **Abstract**

The present invention relates to a method of determining or predicting the sensitivity of a subject to an anti-inflammatory treatment against IBD using vedolizumab, comprising the steps of:
Providing a biological sample of a subject suffering from IBD,
determining the methylation status of at least one CpG located within the nucleotide sequences comprised in the DMRs selected from the group consisting of cg07524919, cg13872627, cg27546431, cg05493407, cg19443707, cg17830959, cg02601475, cg05529343, cg24838063, cg03864958, cg19700328, cg05353869, cg14500486, cg18319102, cg05030953, cg03449857, cg05338672; cg09766721, cg07156742, cg11799593, cgl7096289 and cg09307518, or a nucleotide sequence present within about 2,000bp (such as within about 200bp) 5' or 3' thereof, or an allelic variant and/or complementary sequence of any of said nucleotide sequences, and
determining the sensitivity based on said methylation status wherein a higher level of methylation of cg07524919, cg13872627, cg27546431, cg05493407, cg19443707, cg17830959, cg02601475, cg05529343, cg24838063, cg03864958, cg19700328, cg05353869, cg14500486, cg18319102, cg05030953, cg03449857, cg05338672; and a lower level of methylation of cg09766721, cg07156742, cg11799593, cg17096289, cg09307518 in comparison to a control value or control sample is indicative of an increased sensitivity to a therapy using vedolizumab.

## Description

### TECHNICAL FIELD OF THE INVENTION

The present invention relates in general to the use of the methylation status of particular loci positioned on genomic DNA and their potential to predict drug resistance/susceptibility to the treatment of Crohn's disease. More specifically, the invention relates to the DNA methylation status as biomarkers for predicting the efficacy of treatment using infliximab, vedolizumab and adalimumab in the treatment of CD.

### BACKGROUND OF THE INVENTION

CD is an IBD with an unknown etiology. Based on advancing knowledge of the pathophysiology new therapeutic strategies have been developed that target the pathologic pathways in CD. For over twenty years vedolizumab (VEDO) and other cytokines (IL-12/IL-23) demonstrated efficacy for treating CD. Biomarker discovery for CD based on the personalized genotype, clinical information and immunological reactivity could facilitate the stratification of patients to optimally tailored choice for therapeutic approaches

Despite the proven efficacy of VEDO, only 31% and 36% of Crohn's disease (CD) patients present corticosteroid-free clinical- and endoscopic remission, respectively. Therefore, predictive biomarkers for treatment success would be of extreme value. Previous studies associated differential DNA methylation with CD-specific phenotypes, suggesting a potential use in classification and prediction of response to treatment.

### SUMMARY OF THE INVENTION

The present invention is based on a new panel of epigenetic biomarkers that predict clinical- and endoscopic response in CD patients treated with VEDO. We prospectively measured longitudinal peripheral blood DNA methylation profiles of 93 adult CD patients prior to and following VEDO treatment in a discovery and two separate validation cohorts using the Illumina EPIC BeadChip array. Through classification analysis at time point of starting therapy (T1), we were capable of discriminating R from NR with high predictive performance (AUC 0.89, F1-score of 0.84, PPV of 0.91 and NPV of 0.85).

The invention provides a method of determining or predicting the sensitivity of a subject to an anti-inflammatory treatment against Crohn's disease using vedolizumab, comprising the steps of:
a. providing a biological sample of a subject suffering from Crohn's disease,
b. determining the methylation status of at least one CpG located within the nucleotide sequences comprised in the DMRs selected from the group consisting of cg07524919, cg13872627, cg27546431, cg05493407, cg19443707, cg17830959, cg02601475, cg05529343, cg24838063, cg03864958, cg19700328, cg05353869, cg14500486, cg18319102, cg05030953, cg03449857, cg05338672, cg09766721, cg07156742, cg11799593, cg17096289 and cg09307518 or a nucleotide sequence present within about 2,000bp (such as within about 200bp) 5' or 3' thereof, or an allelic variant and/or complementary sequence of any of said nucleotide sequences,
c. determining the sensitivity based on said methylation status wherein a higher level of methylation of cg07524919, cg13872627, cg27546431, cg05493407, cg19443707, cg17830959, cg02601475, cg05529343, cg24838063, cg03864958, cg19700328, cg05353869, cg14500486, cg18319102, cg05030953, cg03449857, cg05338672; and a lower level of methylation of cg09766721, cg07156742, cg11799593, cg17096289 and cg09307518 in comparison to a control value or control sample is indicative of an increased sensitivity to a therapy using vedolizumab.

Preferably, said biological sample comprises white blood cells. Preferably, said methylation level is determined using DNA methylation array.

The invention further provides Vedolizumab for use in the treatment of a subject who is sensitive to a treatment with vedolizumab as determined using the method according to the invention.

The invention further provides the use of a DNA methylation array for the performing the method according to the invention.

The invention further provides a DNA methylation array suitable for performing the method according to any of claims 1-4 comprising fewer than 1000, preferably fewer than 500, 100, 50 or 25 individual CpG sites.

### BRIEF DESCRIPTION OF THE FIGURES

Figure 1 spider plot depicting 22 DMP most predictive in predicting response to VEDO or no response to VEDO.
Figure 2 shows the receiver operating characteristics (ROC) of predictions model predicting response to treatment using the 22 most predictive markers for VEDO derived from 30 patients ( in a discovery cohort, and validated in 30 patients in a validation cohort.

### DETAILED DESCRIPTION OF THE INVENTION

### Definitions

The term "biological sample" as used herein refers to any sample from an Crohn's disease patient for diagnostic, prognostic, or personalized medicinal uses and may be obtained from surgical samples, such as biopsies or fine needle aspirates, from paraffin-embedded tissues, from a fresh or frozen body fluid. Most preferably the sample contains nucleated blood cells. However, any other suitable biological samples (e.g. bodily fluids such as stool, etc...) in which the methylation status of a locus of interest can be determined are included within the scope of the invention.

By "methylation status" is meant the level of methylation of cytosine residues (found in CpG pairs) in the DNA sequence of interest. When used in reference to a CpG site, the methylation status may be methylated or unmethylated. When used in reference to a CpG island or to any stretch of residues, the methylation status refers to the level of methylation, which is the relative or absolute concentration of methylated C at the particular CpG island or stretch of residues in the DNA present in a biological sample.

The term "hypermethylation" as used herein refers to the average methylation status corresponding to an increased presence of 5-mCyt at one or a plurality of CpG dinucleotides within a DNA sequence of a test DNA sample, relative to the amount of 5-mCyt found at corresponding CpG dinucleotides from a DNA sample from a non-responder. Preferably, a responder is a subject who meets the following criteria at their follow-up endoscopy between 6-24 months after starting biological treatment (if no major medication changes during this period):
a) - ≥50% drop in the endoscopic severity score SES-CD score compared to baseline endoscopy, and
b) - ≥3 point drop in the clinical Harvey Bradshaw index (51) and/or
c) - shows signs of biological improvement based on either a CRP <5.0 mg/l or fecal calprotectin <250ug/g, and
d) - need no steroid treatment at response assessment.

A non- responder is preferably a subject who meets the following criteria at their follow-up endoscopy between 6-24 months after starting biological treatment (if no major medication changes during this period):
a)- <50% drop in SES-CD score compared to baseline endoscopy, and
b) - <3 point drop in Harvey Bradshaw index, and/or
c) - shows no signs of biological improvement based on either an unchanged or increased (delta 25%) CRP and/or fecal calprotectin >250ug/g, and/or
d) - is in need of a steroid treatment at response assessment.

The phrase "corresponding to" when used to describe positions or sites within nucleotide sequences, is used herein as it is understood in the art. As is well known in the art, two or more nucleotide sequences can be aligned using standard bioinformatic tools, including programs such as BLAST, ClustalX, Sequencher, and etc. Even though the two or more sequences may not match exactly and/or do not have the same length, an alignment of the sequences can still be performed and, if desirable, a "consensus" sequence generated. Indeed, programs and algorithms used for alignments typically tolerate definable levels of differences, including insertions, deletions, inversions, polymorphisms, point mutations, etc. Such alignments can aid in the determination of which positions in one nucleotide sequence correspond to which positions in other nucleotide sequences.

The abbreviation "CpG" is used herein to refer to a dinucleotide comprised of a cytosine nucleotide (deoxycytidine) linked via a phosphate group to a guanine nucleotide (deoxyguanosine) through linkages to the 5' position of the deoxycytidine and the 3' position of the deoxyguanosine. The cytosine in this dinucleotide is said to be in the "5' position" of the dinucleotide, and the guanine is said to be in the "3' position" of the dinucleotide. As is understood by one of ordinary skill in the art, the abbreviation "CpG" also refers to modified dinucleotides similar, so long as the 5' nucleotide is still identifiable as deoxycytidine and the 3' nucleotide is still identifiable as deoxyguanosine. For example, a deoxycytidine-deoxyguanosine dinucleotide in which the cytosine ring is methylated at the 5 position is still considered a CpG dinucleotide, and may be referred to as a methylated CpG or abbreviated as 5mCpG. As used herein, the abbreviation CpG can also refer to a CpG site, defined below.

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 kb in length.

The term "Observed/Expected Ratio" ("O/E Ratio") refers to the frequency of CpG dinucleotides within a particular DNA sequence, and corresponds to the (number of CpG sites/(number of C bases X number of G bases)) X band length for each fragment.

The term "CpG site" is used herein to refer to a position within a region of DNA corresponding to a position where a CpG dinucleotide is found in a reference sequence. One of ordinary skill in the art will understand the term CpG site to encompass the location in the region of DNA where a CpG dinucleotide is typically found, whether or not the dinucleotide at that position is a CpG dinucleotide in a particular DNA molecule. For examples, the DNA sequence of a gene may typically contain a CpG dinucleotide at a particular position, but may contain other dinucleotides at the corresponding position in mutant versions, polymorphic variants, or other variations of the gene.

Some mutations such as single base substitutions may alter the identity of the dinucleotide to be something other than CpG. Other mutations such as insertions or deletions may alter the position of the CpG dinucleotide typically found at a particular site. In cases such as these, the term CpG site is understood by one of ordinary skill in the art to encompass the site corresponding to the position where a CpG dinucleotide is typically found, for example, in individuals not carrying a mutation at this location. Similarly, the term CpG site also encompasses the corresponding site in a nucleic acid that has been modified experimentally, for example by labeling, methylation, demethylation, deamination (including conversion of a cytosine to uracil by a chemical such as sodium bisulfite), etc. The CpG loci IDs (cg#) as described herein are as described in Illumina's CpG loci database (Pub. No. 270-2007-006 Current as of 1 February 2008).

The term "predicting the sensitivity to a treatment", as used herein refers to the determination of the likelihood that the patient will respond either favorably or unfavorably to a given therapy. Especially, the term "prediction", as used herein, relates to an individual assessment of any parameter that can be useful in determining the evolution of a patient. Preferred confidence intervals are at least 50%, at least 60%, at least 70%, at least 80%, at least 90% at least 95%. The p- values are, preferably, 0.2, 0.1 or 0.05.

### Detailed description of certain embodiments of the invention

### Methylation assays

The methylation status of a DMR in the DNA of a biological sample may be determined using any methylation assay. Several quantitative methylation assays are commercially available. These include COBRA^{™} (Ziong and Laird, Nucleic Acid Res 1997 25; 2532-4) which uses methylation sensitive restriction endonuclease, gel electrophoresis and detection based on labeled hybridization probes. Another available technique is the Methylation Specific PCR (MSP) for amplification of DNA segments of interest. This is performed after sodium 'bisulfite' conversion of cytosine using methylation sensitive probes. MethyLight^{™}, a quantitative methylation assay-based uses fluorescence based PCR (Eads et al, Cancer Res 1999; 59:2302-2306). Another method used is the Quantitative Methylation (QMTM) assay, which combines PCR amplification with fluorescent probes designed to bind to putative methylation sites. Ms-SNuPE^{™} is a quantitative technique for determining differences in methylation levels in CpG sites. As with other techniques bisulfite treatment is first performed leading to the conversion of unmethylated cytosine to uracil while methyl cytosine is unaffected. PCR primers specific for bisulfite converted DNA is used to amplify the target sequence of interest. The amplified PCR product is isolated and used to quantitate the methylation status of the CpG site of interest (Gonzalgo and Jones Nuclei Acids Res 1997; 25:252-31). The preferred method of measurement of cytosine methylation is the Illumina method.

### Illumina method

For DNA methylation assay the Illumina Infinium^{®} Infinium MethylationEPIC Beadchip assay is preferably used for genome wide quantitative methylation profiling. Briefly genomic DNA is extracted from cells in this case whole blood, for which the original source of the DNA is preferably white blood cells. Using techniques widely known in the trade, the genomic DNA may be isolated using commercial kits. Proteins and other contaminants are preferably removed from the DNA, preferably using proteinase K. The DNA may be removed from the solution using available methods such as organic extraction, salting out or binding the DNA to a solid phase support.

### Bisulfite Conversion

As described in the Infinium^{®} Assay Methylation Protocol Guide, prior to its analysis, DNA must be treated with sodium bisulfite which converts unmethylated cytosine to uracil, while the methylated cytosine remains unchanged. The bisulfite converted DNA is then denatured and neutralized. The genomic DNA may be bisulfite converted using commercial kits, e.g ZYMO^{®}. The denatured DNA is then amplified. The whole genome application process increases the amount of DNA by up to several thousand-fold. The next step uses enzymatic means to fragment the DNA. The fragmented DNA is next precipitated using isopropanol and separated by centrifugation. The separated DNA is next suspended in a hybridization buffer. The fragmented DNA is then hybridized to beads that have been covalently limited to 50 mer nucleotide segments at a locus specific to the cytosine nucleotide of interest in the genome. There are a total of over 850,000 bead types specifically designed to anneal to the locus where the particular cytosine is located. The beads are bound to silicon based arrays. There are two bead types designed for each locus, one bead type represents a probe that is designed to match to the methylated locus at which the cytosine nucleotide will remain unchanged. The other bead type corresponds to an initially unmethylated cytosine which after bisulfite treatment is converted to a thiamine nucleotide. Unhybridized (not annealed to the beads) DNA is washed away leaving only DNA segments bound to the appropriate bead and containing the cytosine of interest. The bead bound oligomer, after annealing to the corresponding patient DNA sequence, then undergoes single base extension with fluorescently labeled nucleotide using the 'overhang' beyond the cytosine of interest in the patient DNA sequence as the template for extension.

If the cytosine of interest is unmethylated then it will match perfectly with the unmethylated or "U" bead probe. This enables single base extensions with fluorescent labeled nucleotide probes and generate fluorescent signals for that bead probe that can be read in an automated fashion. If the cytosine is methylated, single base mismatch will occur with the "U" bead probe oligomer. No further nucleotide extension on the bead oligomer occurs however thus preventing incorporation of the fluorescent tagged nucleotides on the bead. This will lead to low fluorescent signal form the bead "U" bead. The reverse will happen on the "M" or methylated bead probe.

Laser is used to stimulate the fluorophore bound to the single-base used for the sequence extension. The level of methylation at each cytosine locus is determined by the intensity of the fluorescence from the methylated compared to the unmethylated bead. Cytosine methylation level is expressed as "W' which is the ratio of the methylated-bead probe signal to total signal intensity at that cytosine locus. These techniques for determine cytosine methylation have been previously described and are widely available for commercial use.

In preferred embodiments of the invention, the detected methylation status is hypermethylation, an increase in the methylation of cytosines in CpG sites in responders compared to non-responders. The increase can be about 1%, about 5%, about 10%, about 20%, about 25%, about 30%, about 35%, about 40%, about 45%, about 50%, about 60%, about 65%, about 70%, about 75%, about 80%, about 85%, about 90%, about 95%, or more than about 95% greater than the extent of methylation of cytosines typically expected or observed for the CpG site or sites being evaluated. In preferred embodiments of the invention, the detected methylation status is hypomethylation, a decrease in the methylation of cytosines in CpG sites in responders compared to non-responders. The decrease can be about 1%, about 5%, about 10%, about 20%, about 25%, about 30%, about 35%, about 40%, about 45%, about 50%, about 60%, about 65%, about 70%, about 75%, about 80%, about 85%, about 90%, about 95%, or more than about 95% less than the extent of methylation of cytosines typically expected or observed for the CpG site or sites being evaluated. Suitable controls may need to be incorporated in order to ensure the method chosen is working correctly and reliably. Suitable controls may include assessing the methylation status of a gene known to be methylated. This experiment acts as a positive control to ensure that false negative results are not obtained. The DMR may be one which is known to be methylated in the sample under investigation or it may have been artificially methylated. In one embodiment, the DMR of interest may be assessed in normal cells, following treatment with SssI methyltransferase, as a positive control. Additionally or alternatively, suitable negative controls may be employed with the methods of the invention. Here, suitable controls may include assessing the methylation status of a gene known to be unmethylated or a DMR that has been artificially demethylated. This experiment acts as a negative control to ensure that false positive results are not obtained. In one embodiment, the DMR of interest may be assessed in normal cells as a negative control, in particular if the DMR is unmethylated in normal tissues.

In a preferred embodiment, the method according to the invention is determined, by determining if hyper- or hypomethylation is determined in at least two, preferably three, four or five DMRs as disclosed herein. An advantage thereof is that if more of said DMRs are hyper or hypomethylated, the sensitivity of the method is greater.

In certain embodiments of the invention, the detected methylation status is determined in part or wholly on the basis of a comparison with a control. The control can be a value or set of values related to the extent and/or pattern of methylation in a control sample. In certain embodiments of the invention, such a value or values may be determined, for example, by calculations, using algorithms, and/or from previously acquired and/or archived data. In certain embodiments of the invention, the value or set of values for the control is derived from experiments performed on samples or using a subject. For example, control data can be derived from experiments on samples derived from biological samples form subjects known to be sensitive to an anti-inflammatory treatment against IBD, using a monoclonal antibody targeting tumor necrosis factor α or integrin α4β7.

The term "control value" as used herein refers to a value representing the quantity of methylation of said DMR in a non-responder (e.g., from white blood cells from a non-responder), thereby quantifying the average methylation density in the locus compared to the methylation density of the control DNA. A skilled person will understand that it is also possible to compare the methylation of a certain DMR with the methylation level of the same DMR in a responder. In such case, when the methylation status is at a similar level compared to a control representative of a responder (which could be increased or decreased compared to a non-responder), this indicates that a subject is sensitive to a treatment. Using control values of methylation in DMRs of responders is also within the scope of this invention.

In certain embodiments of the invention, a control comprising DNA that is mostly or entirely demethylated, at one or more of the CpG sites being analyzed, is used. Such a control might be obtained, for example, from mutant tissues or cells lacking methyltransferase activity and/or from tissues or cells that have been chemically demethylated. For example, controls may be obtained from tissues or cells lacking activity of methyltransferases Dnmtl, Dnmt2, Dnmt3a, Dnmt3b, or combinations thereof. Agents such as 5-aza-2'-deoxycytidine may be used to chemically demethylate DNA.

In certain embodiments of the invention, a control comprising DNA that is mostly or entirely methylated, at one or more of the CpG sites being analyzed, is used. Such a control might be obtained, for example, from cells or tissues that are known or expected to be mostly or entirely methylated at the CpG site or sites of interest. Such a control could also be obtained by cells or tissues in which methylation levels have been altered and/or manipulated, for example, by overexpression of methyltransferases (such as enzymes Dnmtl, Dnmt2, Dnmt3a, Dnmt3b, any of the bacterial 5-CpG methyltransferases, or combinations thereof). In certain embodiments of the invention, samples used to obtain control values are processed and/or manipulated in the same manner as the samples being evaluated. In a preferred embodiment, said control comprises a colorectal cancer cell line with a known methylation status.

In some embodiments, negative control samples are generated by treating positive control samples with a demethylating agent, preferably 5-aza-2'-deoxycytidine.

### Medical treatment using VEDO

In another aspect, the invention provides VEDO for use in the treatment of a subject who is sensitive to a treatment with VEDO in the treatment as determined using the method according to the invention. If a positive clinical response to treatment with VEDO is determined, the patient is identified or selected for a treatment with VEDO. If a negative clinical response to a treatment is determined, the patient is not selected for treatment, and one or more alternative drug or medical intervention may be more beneficial for the treatment of IBD.

The above disclosure generally describes the present invention. Unless defined otherwise, all technical and scientific terms used herein have the same meaning as those commonly understood by one of ordinary skill in the art to which this invention belongs. A more complete understanding can be obtained by reference to the following specific examples which are provided herein for purposes of illustration only, and are not intended to limit the scope of the invention.

### EXAMPLE SECTION

We prospectively recruited 64 adult CD patients with active clinical- (mean HBI 8), biochemical-(median f. calpro 1154 (IQR 398-1909)) and endoscopic disease activity (median SES-CD 8, (IQR 6-13)) upon a baseline ileocolonoscopy in two separately recruited cohorts (discovery, n=30, 16R/14NR, independent validation, n=34, 20R/14NR). All patients were naive to treatment with VEDO. Forty-nine (77%) patients were previously treated with an anti-TNF and 9 (14%) with Ustekinumab. No significant differences in age, sex and smoking behavior were observed between R and NR in both cohorts (p= 0.98, 0.17 and 0.46 respectively). Responders and non-responders in both cohorts presented overall comparable clinical characteristics yet differences in disease behavior (p=0.00), perianal disease phenotype (p=0.03), previous IBD related surgery (p=0.01) and previous immunomodulatory (p=0.00)- and anti-TNF treatment (p=0.02) could be observed between the discovery and validation datasets (table 1). While non-responders more often received an extra week 10 infusion (35.7% vs 13.9%, p=0.04) during follow-up, the distribution of q8w, q6w or q4w interval was not significantly different between both group at response assessment (p=0.28). Furthermore, all patients had measurable serum VEDO concentrations at response assessment (median 14.5 (6.9 - 21.3) µg/mL) which were comparable between R and NR (median 15 (7.7 - 20.5) vs median 14 (3.6 -27.5), p= 0.77 respectively).

Additional, detailed clinical characteristics of the different cohorts can be found in table 1.

**Table 1 Patient characteristics**

| **Baseline characteristics** | **Vedolizumab discovery R (n=16)** | **Vedolizumab discovery NR (n=14)** | **P-value** | **Vedolizumab validation R (n=20)** | **Vedolizumab validation NR (n=14)** | **P-value** | **Vedolizumab discovery (n=30)** | **Vedolizumab validation (n=34)** | **P-value** |
|---|---|---|---|---|---|---|---|---|---|
| Gender, n (%) | | | | | | | | | |
| - Female | 7 (43.8) | 9 (64.3) | 0.26 | 10 (50) | 9 (64.3) | 0.41 | 16 (53.3) | 19 (55.9) | 0.84 |
| Age, years, median (IQR) | 37 (24-52) | 40 (28-57) | 0.59 | 34 (25-54) | 31 (29-56) | 0.75 | 37 (28-53) | 32 (26-55) | 0.65 |
| Disease duration, years, median (IQR) | 13 (4-18) | 14 (9-22) | 0.29 | 11 (1-23) | 6 (2-14) | 0.67 | 13 (7-20) | 7 (2-19) | 0.08 |
| Ethnic background, n (%) | | | | | | | | | |
| - Caucasian | 13 (81.3) | 12 (85.7) | 0.74 | 16 (80) | 7 (50) | 0.07 | 25 (83.3) | 23 (67.6) | 0.15 |
| C-reactive protein, mg/L, median (IQR) | 3.7 (2.2-12.3) | 6.7 (2.9-25.4) | 0.34 | 4 (1.6-11.4) | 5.5 (3.3-15.6) | 0.54 | 5.1 (2.5-15.2) | 4.6 (2.2-12.2) | 0.62 |
| Faecal calprotectin, ug/g, median (IQR) | 1800 (428.3-1851.3) | 943.5 (336-1800) | 0.64 | 892 (341-1529) | 1880 (763-3142) | 0.10 | 1234 (366-1803) | 1154 (450-2325) | 0.72 |
| Total baseline HBI, median (IQR) | 8 (2-12) | 10 (7-12) | 0.27 | 6 (4-10) | 6 (4-14) | 0.58 | 9 (6-12) | 6 (4-10) | 0.12 |
| Total baseline SES-CD, median (IQR) | 7 (3-10) | 11 (5-17) | 0.05 | 8 (6-15) | 7 (4-12) | 0.25 | 8 (5-12) | 8 (6-13) | 0.92 |
| Disease location, n (%) | | | 0.63 | | | 0.78 | | | 0.90 |
| - Ileal disease (L1) | 5 (31.3) | 4 (28.6) | | 8 (40) | 4 (28.6) | | 9 (30.0) | 12 (35.3) | |
| - Colonic disease (L2) | 5 (31.3) | 2(14.3) | | 4(20) | 3 (21.4) | | 7(23.3) | 7(20.6) | |
| - Ileocolonic disease (L3) | 6(37.5) | 8 (57.1) | | 8 (40) | 7(50) | | 14(46.7) | 14 (44.1) | |
| - Upper GI involvement (L4) | 1 (6.3) | 2 (14.3) | 0.46 | - | - | 1.00 | 3 | - | 0.03 |
| Disease behavior, n (%) | | | 0.38 | | | 0.94 | | | 0.00 |
| - Non structuring non- | 5 (31.3) | 2 (14.3) | | 11 (55) | 7 (50) | | 7 (23.3) | 18 (52.9) | |
| penetrating (B1) | 2 (12.5) | 4 (28.6) | | 8 (40) | 6 (42.9) | | 6 (20.0) | 14 (41.2) | |
| - Stricturing (B2) | 9 (56.3) | 8 (57.1) | | 1 (5) | 1 (7.1) | | 17 (56.7) | 2 (5.9) | |
| - Penetrating (B3) | 6(37.5) | 7(50) | 0.49 | 2(10) | 4(28.6) | 0.16 | 13 (43.3) | 6(17.6) | 0.03 |
| - Perianal disease (p) | | | | | | | | | |
| Previous IBD related surgery (resection, seton, stricturoplasty), n (%) | 7 (43.8) | 12 (85.7) | 0.01 | 7 (35) | 4 (28.6) | 0.69 | 19 (63.3) | 11 (32.4) | 0.01 |
| Concomitant medication, n (%) | 3 (18.8) | 2 (14.3) | 0.46 | 1 (5) | - | 0.30 | 3 (10) | 1 (2.9) | 0.24 |
| - Immunomodulators | | | | | | | | | |
| - Prednisone taper scheme | | | | | | | | | |
| Previous biological agents, n (%) | 16 (100) | 14 (100) | 1.00 | 12 (60) | 10 (71.4) | 0.49 | 30 (100) | 22 (64.7) | 0.00 |
| - Immunomodulators | 14 (87.5) | 13 (92.9) | 0.62 | 10 (50) | 12 (85.7) | 0.03 | 27 (90) | 22 (64.7) | 0.02 |
| - Anti-TNFs | - | 2 (14.3) | 0.08 | 2 (10) | 5 (35.7) | 0.07 | 2 (6.7) | 7 (20.6) | 0.10 |
| - Ustekinumab Smoking, n (%) | | | 0.48 | | | 0.44 | | | 0.85 |
| - Never | 8 (50) | 7 (50) | | 10 (50) | 10 (71.4) | | 15 (50) | 20 (58.8) | |
| -Active | 3 (18.8) | 1(7.1) | | 3 (15) | 1(7.1) | | 4(13.3) | 4(11.8) | |
| - Former | 4 (25) | 6 (42.9) | | 7 (35) | 3 (21.4) | | 10 (33.3) | 10 (29.4) | |
| Serum concentration Vedolizumab at response assessment (ug/mL), median (IQR) | 15 (6-20) | 13.5 (5.7-27.5) | 0.91 | 19 (7.5-24.8) | 17 (3.5-32.5) | 0.84 | 15.5 (7.5-20) | 18 (5.9-26.6) | 0.52 |

**Table 2. Predictor CpG loci**

| **CGID** | **Associated gene** | **Chromosome** | **Position** | **Difference in methylation R vs NR** |
|---|---|---|---|---|
| cg07524919 | TNXB | chr6 | 32063901, exon 3 gene body | Hypermethylation |
| cg13872627 | HLA-C/HLA-B | chr6 | 31238036, exon unknown gene body | Hypermethylation |
| cg27546431 | MYO1C | chr17 | 1372751, intron 25 gene body | Hypermethylation |
| cg05493407 | MARCH1 | chr4 | 164816619, intron 2 gene body | Hypermethylation |
| cg19443707 | | chr8 | 31354807 | Hypermethylation |
| cg09766721 | SLC13A3 | chr20 | 45237014, intron 2-4 gene body | Hypomethylation |
| cg17830959 | HLA-B | chr6 | 31326324, promotor region | Hypermethylation |
| cg02601475 | | chr5 | 121518272 | Hypermethylation |
| cg07156742 | | chr3 | 142666759 | Hypomethylation |
| cg05529343 | ZNF385D | chr3 | 22412124, intron 1 gene body | Hypermethylation |
| cg24838063 | PIWIL1 | chr12 | 130822603, 1^{st} exon | Hypermethylation |
| cg03864958 | | chr4 | 10174168 | Hypermethylation |
| cg19700328 | IGHM | chr14 | 106028568, intron 5063 gene body | Hypermethylation |
| cg05353869 | KLHL35 | chr11 | 75139544, exon 2 ±2000bp from TSS | Hypermethylation |
| cg14500486 | FNDC1 | chr6 | 159655392, exon 9 gene body | Hypermethylation |
| cg18319102 | PIWIL1 | chr12 | 130822256, promotor region | Hypermethylation |
| cg05030953 | HLA-B | chr6 | 31241000, intron 2 and 4 gene body | Hypermethylation |
| cg03449857 | | chr6 | 29648623 | Hypermethylation |
| cg11799593 | | chr12 | 68845869 | Hypomethylation |
| cg17096289 | HLA-C/HLA-B | chr6 | 31238788, introns gene body | Hypomethylation |
| cg05338672 | HLA-C | chr6 | 31241294, intron 4 gene body | Hypermethylation |
| cg09307518 | | chr9 | 38334106 | Hypomethylation |

### Stability selected gradient boosting predicts objective response (leading to remission) to VEDO

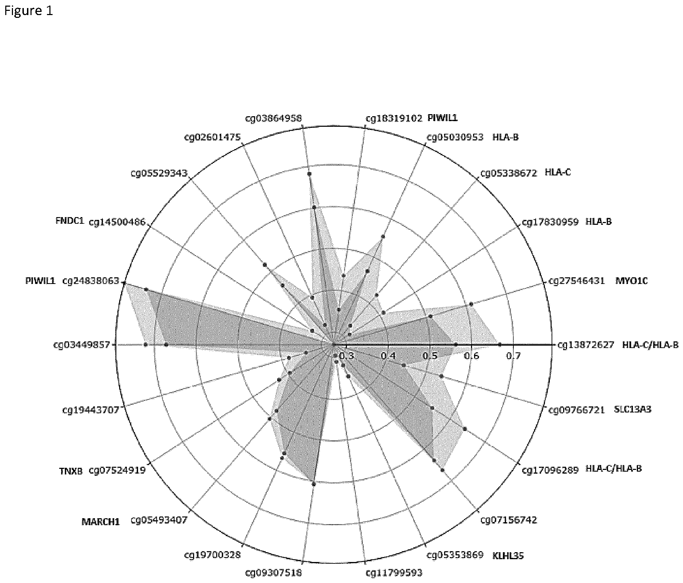

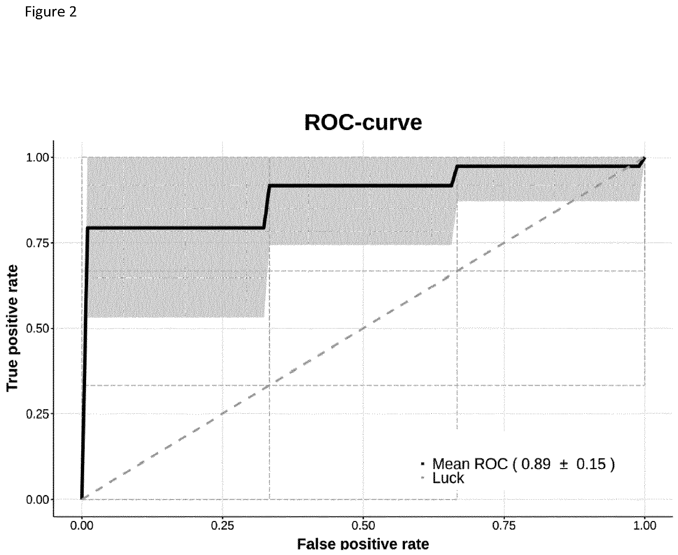

In the Amsterdam discovery cohort (n=30), through stability selected gradient boosting on samples taken prior to start of VEDO treatment (T1), the best predictive model distinguished R from NR with high-accuracy based on a combination of 22 predictor CpG loci (figure 1, 2). Thirteen out of these 22 predictor CpGs annotated to 9 unique genes of which the majority presented hyper methylation (FNDC1, PIWIL1, KLHL35, MARCH1, MYOIC, TNXB, HLA-B or C and ZNF385D) compared to 2 with hypo methylation (SLC13A3 and HLA-B or C) in R vs NR.

We next recalibrated the model based on these 22 CpG loci (see Table 2) after which the performance was tested in the Amsterdam validation cohort (n=34), resulting in an ROC AUC of 0.89 (figure 2).

### Study population and design

We prospectively recruited adult CD patients that presented with active endoscopic disease activity upon baseline ileocolonoscopy and were scheduled to start VEDO treatment in three separately recruited cohorts at the Amsterdam university medical centers (discovery and internal validation cohort) and the John Radcliffe hospital, Oxford (external validation cohort). All patients were naive to treatment with VEDO and were given VEDO at regular dosage of 300mg at week 0, 2 and 6 with subsequent infusions at an 8 week interval, as is standard medical practice. Interval intensification of VEDO to either q6w or q4w as well as an extra week 10 infusion were allowed, if needed, at the treating physicians discretion. To ensure assessment of mechanistic failures to VEDO, only patients with measurable concentrations of VEDO at response assessment were selected.

Additionally, we obtained DNA samples for a larger cohort of CD, and UC patients treated with VEDO from the Oxford Radcliff Hospital as additional, external validation of the 22 markers. The patients response was less strictly determined in this cohort. AUC was 0.80 for 9 CD patients in this cohort, and 0.67 for UC patients in this cohort. So the 22 markers predict response to VEDO in CD patients, rather than UC patients.

### Sample collection and storage protocols

Whole peripheral blood leukocyte (PBL) samples for measurement of epigenome-wide DNA methylation were collected prior to the start of VEDO at either a baseline endoscopy- or week 0 visit (time point 1 [T1 ]) and after a median of 22 weeks into treatment (time point 2 [T2]) using 6.0mL BD ethylenediaminetetraacetic acid (EDTA) vacutainer tubes. For a subset of patients in the Amsterdam discovery cohort (n= 10) a third sample was acquired at a median of 79 weeks into treatment (time point 3 [T3]). Samples were subsequently aliquoted into 1.10mL micronic tubes before storing at -80 °C until further handling. In addition, for a subset of the same patients (n=21), whole peripheral blood samples for transcriptomic analyses were collected using QIAGEN 2.5mL PAXgene Blood RNA tubes at T1 and T2, and stored at -80 °C until further handling.

### Definitions of response and deep remission to VEDO

At response assessment (T2), patients were distinguished as responders (R) or non-responders (NR) based on a strict combination of endoscopic- (≥50% reduction in SES-CD score) combined with either corticosteroid-free clinical- (≥3 point drop87 in HBI or HBI ≤4 and no systemic steroids) and/or biochemical response (C-reactive protein (CRP) and fecal calprotectin reduction ≥50% or ≤5 g/mL and fecal calprotectin ≤250 µg/g).

In a subset of the Amsterdam responding patients (n= 21) we observed deep remission (dR), defined as a combined endoscopic- (SES-CD≤2) and steroid-free clinical remission (HBI ≤4, no systemic steroids) as opposed to the subset of Amsterdam non-responding patients (n=20) that presented with severe non-response (sNR) that presented ≤25% delta change, unchanged or worsened SES-CD and HBI-scores at response assessment.

Two patients without a follow-up endoscopy underwent surgery due to persistent active mucosal inflammation reported upon examination of the resection specimen.

For samples selected out of the Oxford collection, a different, less-strict response definition was used based on a global physicians assessment, better reflecting daily clinical practice.

### DNA isolation and in vitro DNA methylation analysis

For both the discovery and validation cohorts separately, genomic DNA was extracted using the QIAsymphony. We next assessed the quantity of DNA using the FLUOstar OMEGA and quality of the high-molecular weight DNA on a 0.8% agarose gel. Following these steps, 750ng of DNA per sample was randomized per plate, to limit batch effects, after which genomic DNA was bisulfite converted using the Zymo EZ DNA Methylation kit and analyzed on the Illumina HumanMethylation EPIC BeadChip array.

### Raw methylation data pre-processing

Raw methylation data was imported into the R statistical environment using the Bioconductor minfi package (version 1.36), whereupon the raw signals for both the Amsterdam discovery and validation samples were pooled for normalization using a combination of functional and COMBAT normalization to minimize batch effects. We then converted the signals to methylation ratios. The original discovery and validation samples, while normalized together, were separately used for subsequent predictive modelling.

Next, probes binding annotated genetic-variants (min_maf = 0.01) as well as unannotated genetic variants were removed using Gaphunter set to 0.1) in order to increase the identification of true methylation signals rather than genetic variants.

### Machine learning models: stability selected gradient boosting analyses

For both the response- and deep remission model, similar supervised machine learning methodologies were applied. We first split the Amsterdam discovery data into a 70% training- and 30% testing set. Notably, for each biomarker discovery iteration, the model was trained on the 70% training set without exposure to the 30% withheld testing set. Next, we used stability selection with extreme gradient boosting. Gradient boosting is a machine learning technique used for binary classification using a step-wise improvement of tree-based prediction models. Through this step-wise approach, the model builds on existing weaker trees, minimizing the overall prediction error against the observed data. The extreme gradient boosting classification algorithm optimizes a cost function by iteratively choosing a weak hypothesis that points in the negative gradient direction. Ten-fold cross-validation was used to mitigate overfitting which is a common identified problem in machine learning modelling. In addition, to ensure increased confidence in identifying reliable biomarker signals, we used a rigorous stability selection procedure by repeating the above mentioned steps a 100 times, each time completely reshuffling the data and applying a different split in training- and test set.

The resulting many trees (n=100), each containing its own set of ranked CpG markers according to relative importance were then combined using pairwise permutation analysis. This test reruns the model 1000 times, each time randomly removing a CpG and permuting a random variable, assessing with each simulation the effect of this permutation on the model performance (i.e. predicted vs. true outcome). By doing so, this test ranks each CpG of the many identified trees according to its position in a joint panel of selected markers. Therefore only those CpG loci that are ranked above the performance of the permutated random variable, the predictor CpGs, are retained for future analyses. These markers are shown in Table 2.. Finally, as the majority of the CpG loci identified in the many trees mentioned above are not retained due to their rank being below that of the random input variable, we recalibrated the models using only the predictor CpG loci. The resulting calibrated models are then used to distinguish the outcome of interest (response or deep remission) in the Amsterdam/Oxford/UK Biobank validation cohorts after which performance metrics were calculated for each model (n=100 calibrated models). The average combined performance of these models results in the ROC curves shown in figure 2.

Python software was used for the machine learning modelling and visualizations.

### In silico DNA methylation analysis

Differential methylation analyses was performed using limma94 (version 3.46) and eBayes. Statistical significance was defined as a false discovery rate-adjusted p-value < 0.05. Blood cell estimations were performed using the method described by Houseman. Visualizations were generated using ggplot2 version 3.3.5).

### Statistical analysis of clinical variables

Baseline characteristics of all included patients were summarized using descriptive statistics. Categorical variables are presented as percentages and continuous variables as median annotated with the interquartile range (IQR). Differences in distribution between responders, non-responders and the different cohorts were assessed using a chi-square test (categorical variables) or Mann-Whitney U (continuous variables). Two-tailed probabilities were used with a p-value of ≤0.05 considered as statistically significant. Analyses of clinical data were performed in IBM SPSS statistics version 26.

## Claims

1. A method of determining or predicting the sensitivity of a subject to an anti-inflammatory treatment against Crohn's disease using vedolizumab, comprising the steps of:
a. providing a biological sample of a subject suffering from Crohn's disease,
b. determining the methylation status of at least one CpG located within the nucleotide sequences comprised in the DMRs selected from the group consisting of cg07524919, cg13872627, cg27546431, cg05493407, cg19443707, cg17830959, cg02601475, cg05529343, cg24838063, cg03864958, cg19700328, cg05353869, cg14500486, cg18319102, cg05030953, cg03449857, cg05338672, cg09766721, cg07156742, cg11799593, cg17096289 and cg09307518 or a nucleotide sequence present within about 2,000bp (such as within about 200bp) 5' or 3' thereof, or an allelic variant and/or complementary sequence of any of said nucleotide sequences, and
c. determining the sensitivity based on said methylation status wherein a higher level of methylation of cg07524919, cg13872627, cg27546431, cg05493407, cg19443707, cg17830959, cg02601475, cg05529343, cg24838063, cg03864958, cg19700328, cg05353869, cg14500486, cg18319102, cg05030953, cg03449857 and cg05338672; and a lower level of methylation of cg09766721, cg07156742, cg11799593, cg17096289 and cg09307518 in comparison to a control value or control sample is indicative of an increased sensitivity to a therapy using vedolizumab.

2. Method according to claims 1, wherein said biological sample comprises white blood cells.

3. Method according to any of claims 1 - 2, wherein said methylation level is determined using DNA methylation array.

4. Vedolizumab for use in the treatment of a subject who is sensitive to a treatment with vedolizumab as determined using the method according to any of claims 1-3.

5. Use of a DNA methylation array for performing the method according to any of claims 1-4.

6. DNA methylation array or a kit of reagents suitable for performing the method according to any of claims 1-4 comprising probes for detecting fewer than 10000, 5000, 1000, 500, 100, 50 or 25 individual CpG sites.
